# EUROPEAN PATENT APPLICATION

(11) **EP 2 614 774 A1**
(43) Date of publication of application: **17.07.2013**
(21) Application number: 11823327.9
(22) Date of filing: 13.07.2011
(51) Int. Cl.: A61B 8/00

(54) **ULTRASOUND DIAGNOSTIC DEVICE AND METHOD**

(30) Priority: 10.09.2010 JP 2010203073
(71) Applicant: FUJIFILM Corporation, Tokyo 106-8620 (JP)
(72) Inventor: OHSHIMA, Yuji, Ashigara-kami-gun Kanagawa 258-8538 (JP)
(74) Representative: Klunker . Schmitt-Nilson . Hirsch
(86) International application number: PCT/JP2011/065943
(87) International publication number: WO 2012/032848

(57) **Abstract**

Provided is an ultrasound diagnostic device capable of improving the image quality of the region of interest and conserving electric power without limitation of the size of the region of interest. Even if a driving signal is supplied to the various transducers configuring the oscillator array at time (t1), the received signal processor remains OFF and does not turn ON. The received signal processor is only turned ON after the ultrasound echo signal from the region of interest starts being received at time (t4), in accordance with the measurement depth of the region of interest, and until reception of the ultrasound echo signal from the region of interest is completed at time (t5). Thereafter, the received signal processor reverts to the OFF state.

## Description

### TECHNICAL FIELD

The present invention relates to an ultrasound diagnostic device and method, and in particular, to power saving in an ultrasound diagnostic device which is used to perform diagnosis on the basis of an ultrasound image produced through transmission and reception of an ultrasonic wave by means of a transducer array of an ultrasound probe.

### BACKGROUND ART

An ultrasound diagnostic device using an ultrasound image has hitherto been put into practical use in the field of medicine. In general, this type of ultrasound diagnostic apparatus has an ultrasound probe with a built-in transducer array and a diagnostic device body connected to the ultrasound probe. In the ultrasound diagnostic device, an ultrasonic wave is transmitted from the ultrasound probe toward a subject, an ultrasonic echo from the subject is received by the ultrasound probe, and the reception signals are electrically processed in the diagnostic device body to produce an ultrasound image.

In recent years, a portable ultrasound diagnostic device which can be transported to a bed side, an emergency medical scene, or the like and used has been developed. In this portable ultrasound diagnostic device, a battery is used as a power source, and power consumption in the device significantly affects the time of continuous use. If power consumption is high, the amount of heat generation in the device increases, causing an increase in device size due to heat radiation measures and damaging the convenience of a portable type.

In particular, in a wireless probe in which an ultrasound probe and a diagnostic device body are connected together in a wireless manner, or the like, it is necessary to dispose transmission and reception circuits relating to transmission of an ultrasonic wave from a transducer and reception of an ultrasonic echo inside a small probe. This disposition requires significant power saving in these circuits.
On the other hand, in a normal ultrasound diagnostic device, while 50 to 100 V is used as the driving voltage of the transducer, in the wireless probe or the like, the activation voltage of the transducer is kept low due to a restricted mounting space. In order to attain high image quality, it is necessary to increase the S/N of the reception circuit. In general, the S/N of the reception circuit is in relation to power consumption, and the current situation is that it is difficult to reduce power consumption of the reception circuit when obtaining a high S/N.

In regard to power saving in the ultrasound diagnostic device, for example, Patent Literature 1 describes a device in which a bias of a buffer amplifier of a channel uninvolved in reception is stopped. Patent Literature 2 suggests an ultrasound diagnostic device in which the density of measurement lines is increased only in a region of interest while maintaining the number of measurement lines, and the density of measurement lines is decreased in other ranges.

### CITATION LIST

### PATENT LITERATURE

[Patent Literature 1] JP 6-296610 A
[Patent Literature 2] JP 9-192130 A

### SUMMARY OF INVENTION

### TECHNICAL PROBLEMS

However, in the device of Patent Literature 1, since a used channel and an unused channel are distinguished from each other, and the bias of the buffer amplifier of the unused channel is stopped, when attaining power saving, there is a problem in that image quality is degraded.
In the device of Patent Literature 2, since the density of measurement lines is changed to maintain the spatial resolution of the region of interest while maintaining the number of measurement lines of one frame, there is a problem in that image quality becomes uneven in a direction perpendicular to the measurement lines, and the length of the region of interest in this direction becomes restricted.

The invention has been accomplished in order to solve the problems of the related art, and an object of the invention is to provide an ultrasound diagnostic device and method capable of attaining high image quality of a region of interest and power saving without restricting the size of the region of interest.

### SOLUTION TO PROBLEMS

An ultrasound diagnostic device according to the present invention is an ultrasound diagnostic device which transmits an ultrasonic beam from a transducer array of a ultrasound probe toward a subject on the basis of actuation signals supplied from a transmission drive unit, processes reception signals output from the transducer array of the ultrasound probe having received an ultrasonic echo from the subject with a reception signal processor, and produces an ultrasound image on the basis of the processed reception signals using an image producer, the ultrasound diagnostic device comprising control means that controls the reception signal processor such that the reception signal processor is operated in a power saving mode in which processing of the reception signals output from the transducer array is selectively stopped depending on a measurement depth.

Preferably, the control means divides a plurality of measurement lines into a first line group and a second line group, and controls the reception signal processor such that the reception signal processor is operated in a high image quality mode, in which the processing of the reception signals output from the transducer array is performed regardless of the measurement depth, for the measurement lines of the first line group, and is operated in the power saving mode for the measurement lines of the second line group.
In this case, the measurement lines of the first line group and the measurement lines of the second line group may be alternately arranged. Otherwise, it may be constructed such that the first line group has measurement lines arranged in a central portion from among the plurality of measurement lines, and the second line group has measurement lines arranged in both lateral portions of the first line group.

In the power saving mode, the control means may perform the processing of the reception signals output from the transducer array for a region of interest and stop the processing of the reception signals output from the transducer array for other regions.
In this case, the region of interest may be set manually. Also, the ultrasound diagnostic device may further comprise a region of interest selector which automatically selects the region of interest on the basis of brightness from the ultrasound image produced by the image producer. Further, the region of interest may be a peripheral region of a focus point of transmission and reception of ultrasonic waves set in advance.
The control means may control the reception signal processor such that the reception signal processor is operated to selectively switch between a high image quality mode, in which the processing of the reception signals output from the transducer array is performed regardless of the measurement depth, and the power saving mode depending on frame.

An ultrasound diagnostic method according to the present invention is an ultrasound diagnostic method which transmits an ultrasonic beam from a transducer array of a ultrasound probe toward a subject on the basis of actuation signals supplied from a transmission drive unit, processes reception signals output from the transducer array of the ultrasound probe having received an ultrasonic echo from the subject with a reception signal processor, and produces an ultrasound image on the basis of the processed reception signals using an image producer, wherein the reception signal processor is controlled such that the reception signal processor is operated in a power saving mode in which processing of the reception signals output from the transducer array is selectively stopped depending on a measurement depth.

Preferably, a plurality of measurement lines are divided into a first line group and a second line group, and the reception signal processor is controlled such that the reception signal processor is operated in a high image quality mode, in which the processing of the reception signals output from the transducer array is performed regardless of the measurement depth, for the measurement lines of the first line group, and is operated in the power saving mode for the measurement lines of the second line group.
In this case, the measurement lines of the first line group and the measurement lines of the second line group may be alternately arranged. Otherwise, it may be constructed such that the first line group has measurement lines arranged in a central portion from among the plurality of measurement lines, and the second line group has measurement lines arranged in both lateral portions of the first line group.

In the power saving mode, the processing of the reception signals output from the transducer array may be performed for a region of interest, and the processing of the reception signals output from the transducer array may be stopped for other regions.
In this case, the region of interest may be set manually. Also, the region of interest may be automatically selected on the basis of brightness from the ultrasound image produced by the image producer. Further, the region of interest may be a peripheral region of a focus point of transmission and reception of ultrasonic waves set in advance.
The reception signal processor may be controlled such that the reception signal processor is operated to selectively switch between a high image quality mode, in which the processing of the reception signals output from the transducer array is performed regardless of the measurement depth, and the power saving mode depending on frame.

### ADVANTAGEOUS EFFECTS OF INVENTION

According to the invention, the reception signal processor is controlled such that it is operated in the power saving mode in which processing of the reception signal output from the transducer array is selectively stopped depending on the measurement depth, making it possible to attain high image quality of the region of interest and power saving without restricting on the size of the region of interest.

### BRIEF DESCRIPTION OF DRAWINGS

FIG. 1 is a block diagram showing the configuration of an ultrasound diagnostic device according to Embodiment 1 of the invention.
FIG. 2 is a timing chart showing the operation of an actuation signal and a reception signal processor in a high image quality mode.
FIG. 3 is a diagram schematically showing the state of reception signal processing on each measurement line in a high image quality mode.
FIG. 4 is a timing chart showing the operation of an actuation signal and a reception signal processor in Embodiment 1.
FIG. 5 is a diagram schematically showing the state of reception signal processing on each measurement line in Embodiment 1.
FIG. 6 is a diagram schematically showing the state of reception signal processing on each measurement line in Embodiment 2.
FIG. 7 is a diagram schematically showing the state of reception signal processing on each measurement line in a modification of Embodiment 2.
FIG. 8 is a diagram showing a method of setting a region of interest in Embodiment 3.
FIG. 9 is a diagram schematically showing the state of reception signal processing on each measurement line in Embodiment 3.
FIGS. 10A and 10B are diagrams schematically showing the state of reception signal processing on each measurement line in Embodiment 4, and specifically, FIG. 10A shows an odd-numbered frame and FIG. 10B shows an even-numbered frame.
FIGS. 11A and 11B are diagrams schematically showing the state of reception signal processing on each measurement line in a modification of Embodiment 4, and specifically, FIG. 11A shows an odd-numbered frame and FIG. 11B shows an even-numbered frame.
FIGS. 12A and 12B are diagrams schematically showing the state of reception signal processing on each measurement line in another modification of Embodiment 4, and specifically, FIG. 12A shows an odd-numbered frame and FIG. 12B shows an even-numbered frame.
FIG. 13 is a block diagram showing the configuration of an ultrasound diagnostic device according to Embodiment 5.
FIG. 14 is a diagram showing a method of discriminating a region of interest in Embodiment 5.

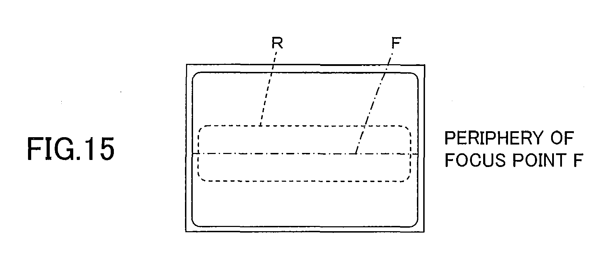

FIG. 15 is a diagram showing a method of setting a region of interest in Embodiment 6.

### DESCRIPTION OF EMBODIMENTS

Hereinafter, embodiments of the invention will be described with reference to the accompanying drawings.

### Embodiment 1

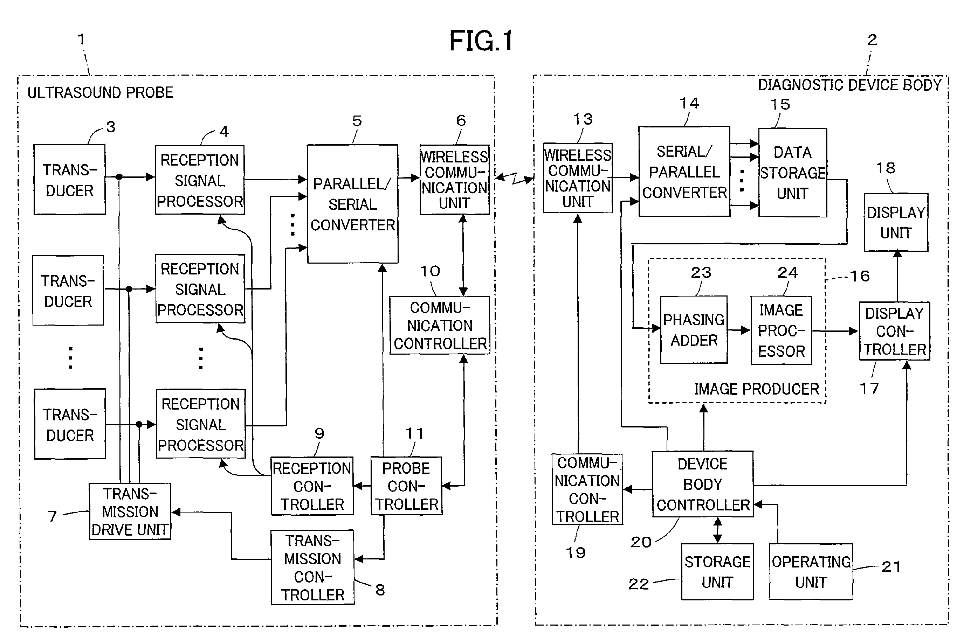

Fig. 1 illustrates the configuration of an ultrasound diagnostic device according to Embodiment 1 of the invention. The ultrasound diagnostic device includes a rechargeable ultrasound probe 1 and a diagnostic device body 2 connected to the ultrasound probe 1 through wireless communication.

The ultrasound probe 1 has a plurality of ultrasound transducers 3 which constitute a one-dimensional or two-dimensional transducer array. Reception signal processors 4 are correspondingly connected to the transducers 3, and a wireless communication unit 6 is connected to the reception signal processors 4 through a parallel/serial converter 5. A transmission controller 8 is connected to the plurality of transducers 3 through a transmission drive unit 7, a reception controller 9 is connected to the plurality of reception signal processors 4, and a communication controller 10'is connected to the wireless communication unit 6. A probe controller 11 is connected to the parallel/serial converter 5, the transmission controller 8, the reception controller 9, and the communication controller 10.

Each of the plurality of transducers 3 forms a vibrator. Each transducer 3 transmits an ultrasonic wave in response to an actuation signal supplied from the transmission drive unit 7 and receives an ultrasonic echo from the subject, and outputs a reception signal. Each transducer 3 is constituted by a vibrator in which electrodes are formed at both ends of a piezoelectric body composed of, for example, piezoelectric ceramic represented by PZT (lead zirconate titanate), a polymer piezoelectric device represented by PVDF (polyvinylidene fluoride), or the like.
If a pulsed or continuous-wave voltage is applied across the electrodes of the vibrator, the piezoelectric body expands and contracts, whereby pulsed or continuous-wave ultrasonic waves are produced from the respective vibrators and the produced ultrasonic waves are synthesized to form an ultrasonic beam. When receiving propagating ultrasonic waves, the respective vibrators expand and contract to produce electric signals, and the electric signals are output as the reception signals of the ultrasonic waves.

The transmission drive unit 7 includes, for example, a plurality of pulse generators. The transmission drive unit 7 adjusts the delay amount of each of the actuation signals on the basis of a transmission delay pattern selected by the transmission controller 8 such that the ultrasonic waves transmitted from the plurality of transducers 3 form an ultrasonic beam having a width enough to cover the area of a tissue in the subject, and supplies the adjusted actuation signals to the plurality of transducers 3.

The reception signal processor 4 of each channel performs quadrature detection processing or quadrature sampling processing on the reception signal output from the corresponding transducer 3 under the control of the reception controller 9 to produce a complex baseband signal, samples the complex baseband signal to produce sample data including information on the area of the tissue, and supplies the sample data to the parallel/serial converter 5. The reception signal processor 4 may perform data compression processing for low bit rate coding on data obtained by sampling the complex baseband signal to produce sample data.
The parallel/serial converter 5 converts parallel sample data produced by the plurality of channels of reception signal processors 4 to serial sample data.

The wireless communication unit 6 modulates a carrier on the basis of the serial sample data to produce a transmission signal, supplies the transmission signal to an antenna, and transmits a radio wave from the antenna, thereby transmitting the serial sample data. As the modulation system, for example, ASK (Amplitude Shift Keying), PSK (Phase Shift Keying), QPSK (Quadrature Phase Shift Keying), 16QAM (16 Quadrature Amplitude Modulation), or the like is used.
The wireless communication unit 6 performs wireless communication with the diagnostic device body 2 to transmit the sample data to the diagnostic device body 2 and to receive various control signals from the diagnostic device body 2, and outputs the received control signals to the communication controller 10. The communication controller 10 controls the wireless communication unit 6 such that the sample data is transmitted with transmission radio field intensity set by the probe controller 11, and outputs various control signals received by the wireless communication unit 6 to the probe controller 11.

The probe controller 11 controls the respective units of the ultrasound probe 1 on the basis of various control signals transmitted from the diagnostic device body 2.
The ultrasound probe 1 has a battery built-in not shown in Fig. 1, and the battery supplies power to the respective circuits in the ultrasound probe 1.
The ultrasound probe 1 may be an external probe, such as a linear scan type, a convex scan type, or a sector scan type, or may be a probe for an ultrasound endoscope, such as a radial scan type.

The diagnostic device body 2 has a wireless communication unit 13. A data storage unit 15 is connected to the wireless communication unit 13 through a serial/parallel converter 14, and an image producer 16 is connected to the data storage unit 15. A display unit 18 is connected to the image producer 16 through a display controller 17. A communication controller 19 is connected to the wireless communication unit 13, and a device body controller 20 is connected to the serial/parallel converter 14, the image producer 16, the display controller 17, and the communication controller 19. An operating unit 21 for an input operation of an operator and a storage unit 22 which stores operation programs are respectively connected to the device body controller 20.

The wireless communication unit 13 performs wireless communication with the ultrasound probe 1 to transmit various control signals to the ultrasound probe 1. Besides, the wireless communication unit 13 demodulates a signal received by an antenna to output serial sample data.
The communication controller 19 controls the wireless communication unit 13 such that various control signals are transmitted with transmission radio field intensity set by the device body controller 20.
The serial/parallel converter 14 converts the serial sample data output from the wireless communication unit 13 to parallel sample data. The data storage unit 15 is constituted by a memory, a hard disk, or the like, and stores the sample data for at least one frame converted by the serial/parallel converter 14.

The image producer 16 performs reception focus processing on the sample data for every frame read from the data storage unit 15 to produce an image signal representing an ultrasound diagnostic image. The image producer 16 includes a phasing adder 23 and an image processor 24.
The phasing adder 23 performs the reception focus processing by selecting one reception delay pattern from among a plurality of reception delay patterns stored in advance in accordance with a reception direction set in the device body controller 20, giving a delay to each of a plurality of complex baseband signals represented by the sample data on the basis of the selected reception delay pattern, and adding the reception signals. With this reception focus processing, the focus of the ultrasonic echo is narrowed to produce a baseband signal (sound ray signal).

The image processor 24 produces a B-mode image signal, which is tomographic image information relating to a tissue in the subject, on the basis of the sound ray signal produced by the phasing adder 23. The image processor 24 includes an STC (sensitivity time control) unit and a DSC (digital scan converter). The STC unit corrects attenuation depending on the distance in accordance with the depth of the reflection position of the ultrasonic wave for the sound ray signal. The DSC converts (raster-converts) the sound ray signal corrected by the STC unit to an image signal based on a normal television signal scan system, and performs necessary image processing, such as gradation processing, to produce a B-mode image signal.

The display controller 17 displays an ultrasound diagnostic image on the display unit 18 on the basis of the image signal produced by the image producer 16. The display unit 18 includes, for example, a display device, such as an LCD, and displays the ultrasound diagnostic image under the control of the display controller 17.

On the basis of the measurement depth of the region of interest input from the operating unit 21 by the operator, the device body controller 20 performs control such that the reception signal processor 4 of the ultrasound probe 1 is operated in the power saving mode in which the reception signal processor 4 is selectively stopped depending on the measurement depth. That is, the device body controller 20 controls the reception signal processor 4 of the ultrasound probe 1 through wireless communication such that the reception signal processor 4 is ON for the measurement depth of the region of interest input from the operating unit 21 and OFF for other measurement depths.

Although in the diagnostic device body 2, the serial/parallel converter 14, the image producer 16, the display controller 17, the communication controller 19, and the device body controller 20 are constituted by a CPU and operation programs which cause the CPU to perform various types of processing, these may be constituted by digital circuits. The operation programs are stored in the storage unit 22. As a recording medium in the storage unit 22, in addition to an internal hard disk, a flexible disk, an MO, an MT, a RAM, a CD-ROM, a DVD-ROM, or the like may be used.

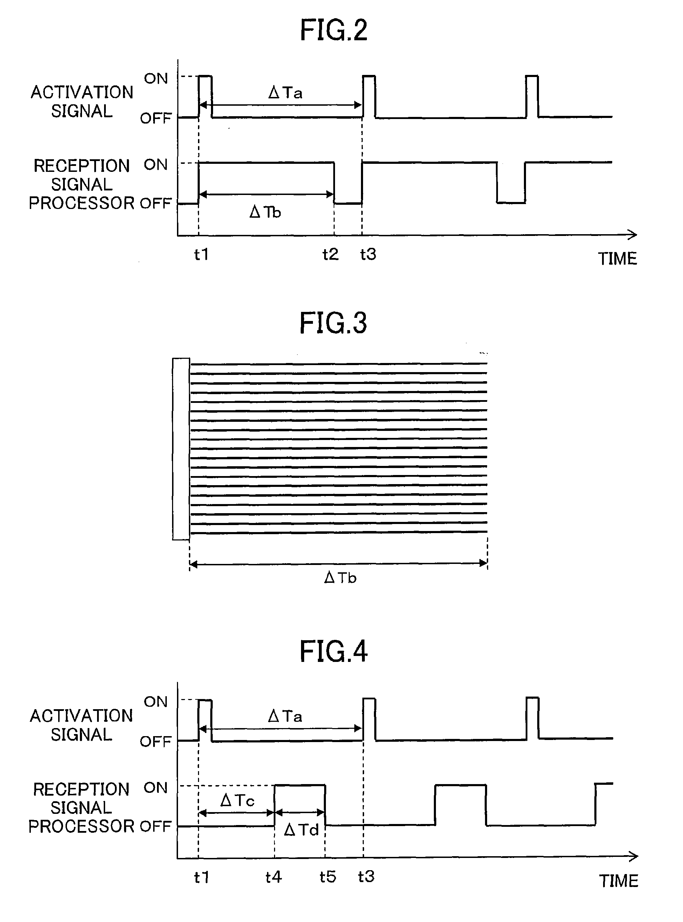

Hereinafter, the power saving mode will be described, but before the explanation of the power saving mode, the high image quality mode in which power saving will not be attained will be described.
In the high image quality mode, as shown in FIG. 2, when the actuation signal is supplied from the transmission drive unit 7 to each transducer 3 constituting the transducer array in a period ΔTa, the reception signal processor 4 is turned on for the time ΔTb sufficient for receiving an ultrasonic echo from the deepest portion of the region to be examined of the subject. For example, if the actuation signal is supplied to the transducer 3 at the time t1, the corresponding reception signal processor 4 enters the ON state at the same time, the ON state of the reception signal processor 4 is maintained over the time ΔTb, and when the reception of the ultrasonic echo effective for examination is completed at the time t2 when the time ΔTb has elapsed from the time t1, the reception signal processor 4 enters the OFF state. Thereafter, the next actuation signal is supplied to the transducer 3 at the time t3 when the period ΔTa has elapsed from the time t1, and the reception signal processor 4 enters the ON state again. Subsequently, the same operation is repeated.

Accordingly, as shown in FIG. 3, for all measurement lines, the processing of the reception signal is performed over the time ΔTb sufficient for receiving the ultrasonic echo from the deepest portion of the region to be examined using the reception signal processor 4, thereby obtaining a high-quality image.

Meanwhile, in the power saving mode, as shown in FIG. 4, when the actuation signal is supplied from the transmission drive unit 7 to each transducer 3 constituting the transducer array in the period ΔTa, the reception signal processor 4 enters the ON state only for the time corresponding to a specific measurement depth of the region of interest input from the operating unit 21, and the reception signal processor 4 enters the OFF state otherwise. For example, even when the actuation signal is supplied to the transducer 3 at the time t1, the reception signal processor 4 does not yet enter the ON state, and enters the ON state at the time t4 when the time ΔTc has elapsed from the time t1. The time ΔTc is the time needed until the reception of the ultrasonic echo from the region of interest starts after the ultrasonic wave is transmitted. That is, the reception signal processor 4 enters the ON state at the time t4 when the reception of the ultrasonic echo from the region of interest starts, and the ON state of the reception signal processor 4 is maintained over the time ΔTd up to the time t5 when the reception of the ultrasonic echo from the region of interest ends. When the reception of the ultrasonic echo from the entire region of interest is completed at the time t5, the reception signal processor 4 enters the OFF state, and thereafter, the next actuation signal is supplied to the transducer 3 at the time t3 when the period ΔTa has elapsed from the time t1. Subsequently, the same operation is repeated.

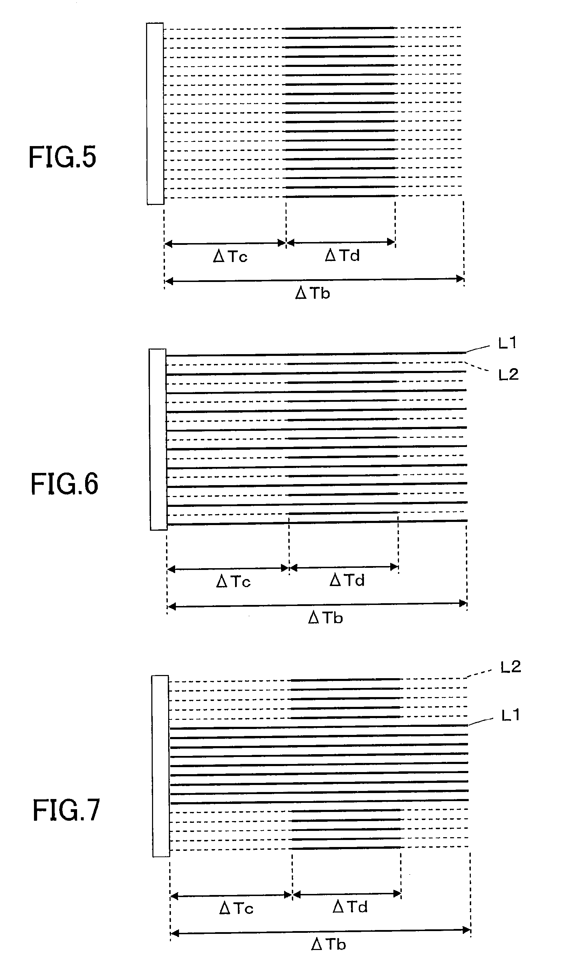

Accordingly, as shown in FIG. 5, for all measurement lines, the processing of the reception signal is performed only for the time ΔTd necessary for receiving the ultrasonic echo from the region of interest using the reception signal processor 4, and the processing of the reception signal is stopped otherwise. As a result, it is possible to attain power saving.

Next, the operation of Embodiment 1 will be described.
First, the operator inputs the measurement depth of the region of interest of the subject from the operating unit 21. For example, the depth from the body surface of the subject can be roughly recognized depending on a portion to be examined or the like, and the measurement depth of the region of interest can be estimated and input on the basis of the depth. The measurement depth of the region of interest input from the operating unit 21 is transmitted from the device body controller 20 of the diagnostic device body 2 to the probe controller 11 of the ultrasound probe 1 through wireless communication.

If ultrasound diagnosis starts, ultrasonic waves are transmitted from a plurality of transducers 3 constituting the transducer array in accordance with the actuation signals supplied from the transmission drive unit 7 under the control of the probe controller 11 and the transmission controller 8. The reception signal output from each transducer 3 having received the ultrasonic echo from the subject is supplied to the corresponding reception signal processor 4. At this time, the probe controller 11 controls the reception signal processor 4 through the reception controller 9 such that the reception signal processor 4 enters the ON state for the measurement depth of the region of interest and enters the OFF state for other measurement depths.

Accordingly, sample data is produced only for the measurement depth of the region of interest using the reception signal processor 4, is serialized using the parallel/serial converter 5, and is transmitted from the wireless communication unit 6 to the diagnostic device body 2 in a wireless manner. The sample data received by the wireless communication unit 13 of the diagnostic device body 2 is converted to parallel data using the serial/parallel converter 14, and is stored in the data storage unit 15. The sample data for one frame is read from the data storage unit 15, an image signal is produced using the image producer 16, and an ultrasound diagnostic image is displayed on the display unit 18 on the basis of the image signal by the display controller 17.

In this way, the reception signal processor 4 of the ultrasound probe 1 is operated in the power saving mode, making it possible to attain power saving while displaying the image of the region of interest with high image quality.
The number of measurement lines is not limited, and the reception signal processor 4 enters the OFF state only for the measurement depth independent of the region of interest. Therefore, it is possible to attain both high image quality of the region of interest and power saving without restricting the size of the region of interest.

### Embodiment 2

Although in Embodiment 1, for all measurement lines, the processing of the reception signal is performed only for the time ΔTd corresponding to the measurement depth of the region of interest using the reception signal processor 4, and the processing of the reception signal is stopped otherwise, the invention is not limited thereto. The measurement lines may be divided into a first line group and a second line group, and the reception signal processor 4 may be controlled such that it is operated in the high image quality mode, in which the processing of the reception signal is performed regardless of the measurement depth, for the measurement lines of the first line group, and is operated in the power saving mode, in which the processing of the reception signal is performed only for the measurement depth of the region of interest, for the measurement lines of the second line group.

For example, as shown in FIG. 6, the setting is made such that odd-numbered lines from among a plurality of measurement lines are grouped in a first line group L1, even-numbered lines are grouped in a second line group L2, and the measurement lines of the first line group L1 and the measurement lines of the second line group L2 are alternately arranged. Then, the reception signal processor 4 is controlled such that it is operated in the high image quality mode for the measurement lines of the first line group L1, and is operated in the power saving mode for the measurement lines of the second line group L2. That is, the reception signal is processed over the time ΔTb sufficient for receiving the ultrasonic echo from the deepest portion of the region to be examined of the subject in the measurement lines of the first line group L1, and the reception signal is processed only for the time ΔTd necessary for receiving the ultrasonic echo from the region of interest in the measurement lines of the second line group L2.

With this, it becomes possible to obtain an ultrasound image over the entire screen and to display the image of the measurement depth of the region of interest with high image quality.
The reception signal processor 4 may be controlled such that it is operated in the high image quality mode for a first line group L1 having even-numbered lines, and is operated in the power saving mode for a second line group L2 having odd-numbered lines.
Moreover, instead of grouping each measurement line in the first line group L1 or the second line group L2, several measurement lines may be grouped in the first line group L1 or the second line group L2.

Furthermore, as shown in FIG. 7, the reception signal processor 4 may be controlled such that it is operated in the high image quality mode for a first line group L1 having a plurality of measurement lines arranged in a central portion from among a plurality of measurement lines, and is operated in the power saving mode for a second line group L2 having a plurality of measurement lines arranged in both lateral portions of the first line group L1.
Thereby, it becomes possible to perform image display with high image quality only for the measurement depths of the central portion and the region of interest while attaining power saving.

### Embodiment 3

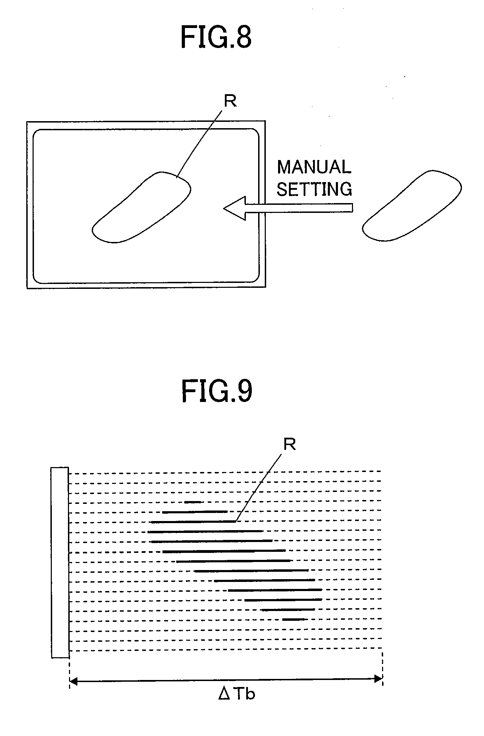

Although in Embodiments 1 and 2 described above, the processing of the reception signal is performed for all measurement lines on the basis of the measurement depth of the region of interest, as shown in FIG. 8, if the operator manually inputs and sets not only the measurement depth of the region R of interest but also the shape of the region R of interest from the display panel of the display unit 18, the operating unit 21, or the like, as shown in FIG. 9, the processing of the reception signal may be performed only for the region R of interest. That is, the processing of the reception signal is performed in the measurement lines where there is the region R of interest for the measurement depth where there is the region R of interest, and the processing of the reception signal is stopped for other regions.
With this, it is possible to attain power saving more effectively.

### Embodiment 4

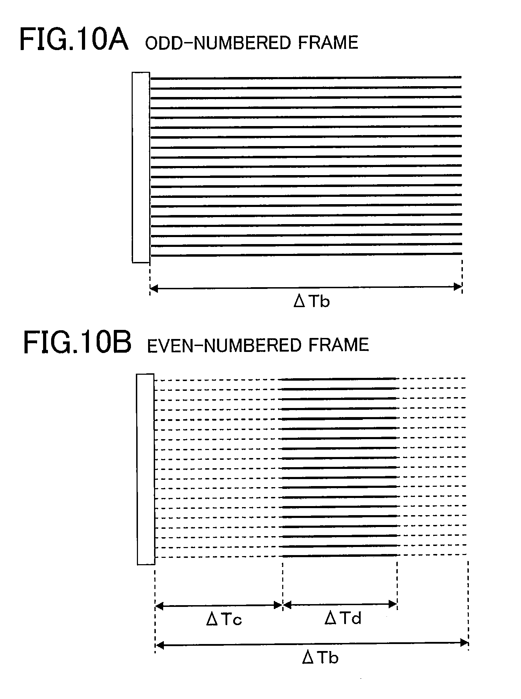

Although in Embodiments 1 to 3 described above, the reception signal processor 4 is controlled without changing depending on frame, the invention is not limited thereto, and the reception signal processor 4 may be controlled such that it is operated to selectively switch between the high image quality mode and the power saving mode depending on frame.
For example, the reception signal processor 4 is controlled such that, as shown in FIG. 10A, it is operated in the high image quality mode, in which the reception signal is processed for all measurement lines regardless of the measurement depth, in the odd-numbered frames, and as shown in FIG. 10B, the processing of the reception signal is performed only for the time ΔTd necessary for receiving the ultrasonic echo from the region of interest and is stopped otherwise for all measurement lines in the even-numbered frames.

With this, it becomes possible to obtain an ultrasound image over the entire screen for each frame and to perform image display with high image quality only for the measurement depth of the region of interest.
The reception signal processor 4 may be controlled such that the even-numbered frame is operated in the high image quality mode and the odd-numbered frame is operated in the power saving mode.
Moreover, the high image quality mode and the power saving mode may be switched for every several frames, instead of switching between the high image quality mode and the power saving mode for every frame.

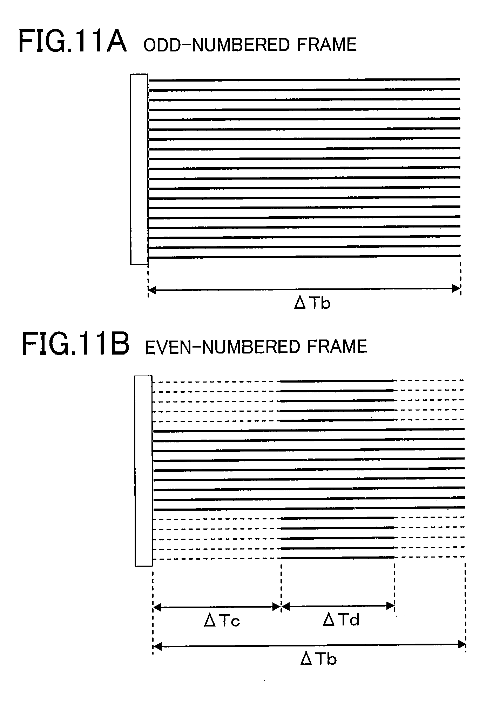

Alternatively, the reception signal processor 4 may be controlled such that, as shown in FIG. 11A, the reception signal processor 4 is operated in the high image quality mode, in which the reception signal is processed for all measurement lines regardless of the measurement depth, in the odd-numbered frames, and as shown in FIG. 11B, the reception signal processor 4 is operated in the high image quality mode for a plurality of measurement lines arranged in the center portion from among a plurality of measurement lines and is operated in the power saving mode for a plurality of measurement lines arranged in both lateral portions in the even-numbered frame.

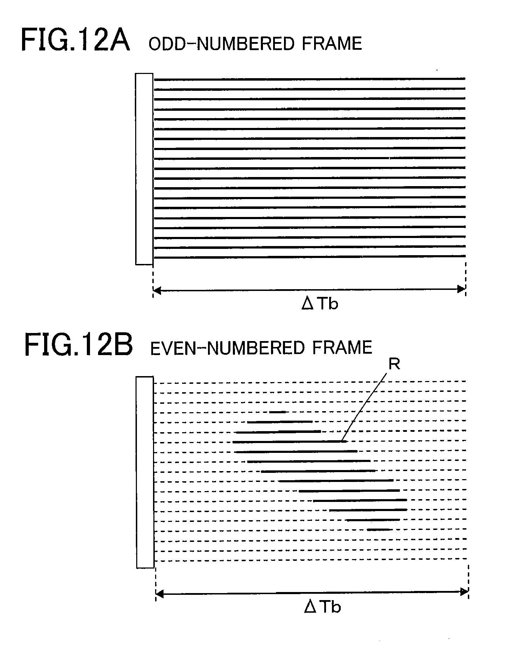

Furthermore, as in Embodiment 3, the operator may manually input and set not only the measurement depth of the region R of interest but also the shape of the region R of interest from the display panel of the display unit 18, the operating unit 21, or the like, and the reception signal processor 4 may be controlled such that it is operated in the high image quality mode, in which the reception signal is performed for all measurement lines regardless of the measurement depth, in the odd-numbered frame as shown in FIG. 12A and performs the processing of the reception signal only for the region R of interest in the even-numbered frame as shown in FIG. 12B.

### Embodiment 5

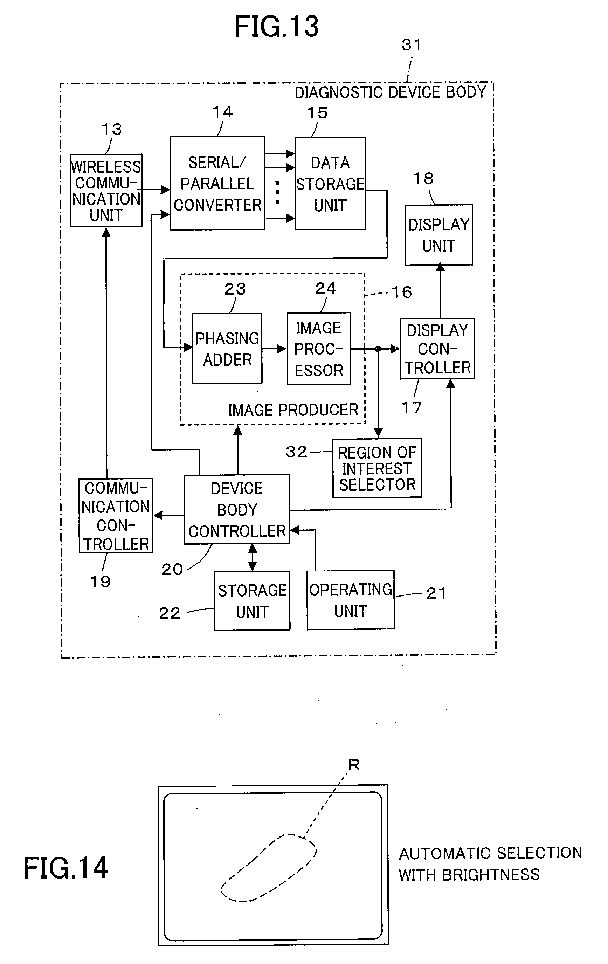

FIG. 13 shows the configuration of a diagnostic device body 31 which is used in an ultrasound diagnostic device according to Embodiment 5. A diagnostic device body 31 is configured such that a region of interest selector 32 is connected to the image producer 16, and the device body controller 20 is connected to the region of interest selector 32, compared to the diagnostic device body 2 of Embodiment 1 shown in FIG. 1. As shown in FIG. 14, the region of interest selector 32 automatically selects the region R of interest on the basis of brightness from the ultrasound image produced by the image producer 16.

With the region of interest selector 32, the region R of interest is automatically selected on the basis of brightness from the ultrasound image produced by the image producer 16. The device body controller 20 controls the reception signal processor 4 through wireless communication such that, as in Embodiment 3, the reception signal processor 4 of the ultrasound probe 1 performs the processing of the reception signal only for the region R of interest on the basis of the region R of interest automatically selected by the region of interest selector 32 and stops the processing of the reception signal for other regions.
As in Embodiment 4 of FIG. 12, the device body controller 20 may control the reception signal processor 4 such that it switches the operation in the high image quality mode and the operation in the power saving mode, in which the processing of the reception signal is performed only for the region R of interest, depending on the frame.

### Embodiment 6

In the diagnostic device body 2 of Embodiment 1 shown in FIG. 1, the device body controller 20 may automatically select a peripheral region of a focus point F of transmission and reception of an ultrasonic wave set in advance as the region R of interest. The device body controller 20 controls the reception signal processor 4 on the basis of the region R of interest automatically selected in this way as described in Embodiments 1 to 4.

Although in Embodiments 1 to 6 described above, the ultrasound probe 1 and the diagnostic device body 2 or 31 are connected together in a wireless manner, the invention is not limited thereto, and the ultrasound probe 1 may be connected to the diagnostic device body 2 or 31 through a connection cable. In this case, the wireless communication unit 6 and the communication controller 10 of the ultrasound probe 1, the wireless communication unit 13 and the communication controller 19 of the diagnostic device body 2 or 31, and the like are not required.

### REFERENCE SIGNS LIST

1 ultrasound probe; 2, 31 diagnostic device body; 3 transducer; 4 reception signal processor; 5 parallel/serial converter; 6 wireless communication unit; 7 transmission drive unit; 8 transmission controller; 9 reception controller; 10 communication controller; 11 probe controller; 13 wireless communication unit; 14 serial/parallel converter; 15 data storage unit; 16 image producer; 17 display controller; 18 display unit; 19 communication controller; 20 device body controller; 21 operating unit; 22 storage unit; 23 phasing adder; 24 image processor; 32 region of interest selector; L1 first line group; L2 second line group; R region of interest; F focus point.

## Claims

1. An ultrasound diagnostic device which transmits an ultrasonic beam from a transducer array of a ultrasound probe toward a subject on the basis of actuation signals supplied from a transmission drive unit, processes reception signals output from the transducer array of the ultrasound probe having received an ultrasonic echo from the subject with a reception signal processor, and produces an ultrasound image on the basis of the processed reception signals using an image producer, the ultrasound diagnostic device comprising:
control means that controls the reception signal processor such that the reception signal processor is operated in a power saving mode in which processing of the reception signals output from the transducer array is selectively stopped depending on a measurement depth.

2. The ultrasound diagnostic device according to claim 1, wherein the control means divides a plurality of measurement lines into a first line group and a second line group, and controls the reception signal processor such that the reception signal processor is operated in a high image quality mode, in which the processing of the reception signals output from the transducer array is performed regardless of the measurement depth, for the measurement lines of the first line group, and is operated in the power saving mode for the measurement lines of the second line group.

3. The ultrasound diagnostic device according to claim 2, wherein the measurement lines of the first line group and the measurement lines of the second line group are alternately arranged.

4. The ultrasound diagnostic device according to claim 2, wherein the first line group has measurement lines arranged in a central portion from among the plurality of measurement lines, and the second line group has measurement lines arranged in both lateral portions of the first line group.

5. The ultrasound diagnostic device according to claim 1, wherein, in the power saving mode, the control means performs the processing of the reception signals output from the transducer array for a region of interest and stops the processing of the reception signals output from the transducer array for other regions.

6. The ultrasound diagnostic device according to claim 5, wherein the region of interest is set manually.

7. The ultrasound diagnostic device according to claim 5, further comprising:
a region of interest selector which automatically selects the region of interest on the basis of brightness from the ultrasound image produced by the image producer.

8. The ultrasound diagnostic device according to claim 5, wherein the region of interest is a peripheral region of a focus point of transmission and reception of ultrasonic waves set in advance.

9. The ultrasound diagnostic device according to any one of claims 1 to 8,
wherein the control means controls the reception signal processor such that the reception signal processor is operated to selectively switch between a high image quality mode, in which the processing of the reception signals output from the transducer array is performed regardless of the measurement depth, and the power saving mode depending on frame.

10. An ultrasound diagnostic method which transmits an ultrasonic beam from a transducer array of a ultrasound probe toward a subject on the basis of actuation signals supplied from a transmission drive unit, processes reception signals output from the transducer array of the ultrasound probe having received an ultrasonic echo from the subject with a reception signal processor, and produces an ultrasound image on the basis of the processed reception signals using an image producer,
wherein the reception signal processor is controlled such that the reception signal processor is operated in a power saving mode in which processing of the reception signals output from the transducer array is selectively stopped depending on a measurement depth.

11. The ultrasound diagnostic method according to claim 10, wherein a plurality of measurement lines are divided into a first line group and a second line group, and the reception signal processor is controlled such that the reception signal processor is operated in a high image quality mode, in which the processing of the reception signals output from the transducer array is performed regardless of the measurement depth, for the measurement lines of the first line group, and is operated in the power saving mode for the measurement lines of the second line group.

12. The ultrasound diagnostic method according to claim 11, wherein the measurement lines of the first line group and the measurement lines of the second line group are alternately arranged.

13. The ultrasound diagnostic method according to claim 11, wherein the first line group has measurement lines arranged in a central portion from among the plurality of measurement lines, and the second line group has measurement lines arranged in both lateral portions of the first line group.

14. The ultrasound diagnostic method according to claim 10, wherein, in the power saving mode, the processing of the reception signals output from the transducer array is performed for a region of interest, and the processing of the reception signals output from the transducer array is stopped for other regions.

15. The ultrasound diagnostic method according to claim 14, wherein the region of interest is set manually.

16. The ultrasound diagnostic method according to claim 14, wherein the region of interest is automatically selected on the basis of brightness from the ultrasound image produced by the image producer.

17. The ultrasound diagnostic method according to claim 14, wherein the region of interest is a peripheral region of a focus point of transmission and reception of ultrasonic waves set in advance.

18. The ultrasound diagnostic method according to any one of claims 10 to 17,
wherein the reception signal processor is controlled such that the reception signal processor is operated to selectively switch between a high image quality mode, in which the processing of the reception signals output from the transducer array is performed regardless of the measurement depth, and the power saving mode depending on frame.
